**Europäisches Patentamt**

**European Patent Office**

**Office européen des brevets**

(19)

(11) Veröffentlichungsnummer: **0 024 028**
**B1**

(12) **EUROPÄISCHE PATENTSCHRIFT**

(45) Veröffentlichungstag der Patentschrift:
**20.07.83**

(51) Int. Cl.³: **A 61 B 6/02**, A 61 B 6/06

(21) Anmeldenummer: **80104600.4**

(22) Anmeldetag: **04.08.80**

(54) **Röntgengerät zur Herstellung von Transversalschichtbildern und Röntgenschattenbildern eines Aufnahmeobjektes.**

(30) Priorität: **08.08.79 DE 2932182**

(43) Veröffentlichungstag der Anmeldung:
**18.02.81 Patentblatt 81/7**

(45) Bekanntmachung des Hinweises auf die Patenterteilung:
**20.07.83 Patentblatt 83/29**

(84) Benannte Vertragsstaaten:
**FR GB**

(56) Entgegenhaltungen:
**DE-A-1 941 433**
**DE-A-2 452 166**
**DE-A-2 613 809**
**DE-A-2 614 083**
**FR-A-2 352 296**
**FR-A-2 400 717**
**GB-A-1 462 861**
**US-A-4 196 352**

(73) Patentinhaber: **SIEMENS AKTIENGESELLSCHAFT,
Berlin und München Wittelsbacherplatz 2,
D-8000 München 2 (DE)**

(72) Erfinder: **Pfeiler, Manfred, Dr. Dr.-Ing.,
Ludwig-Thoma-Strasse 25, D-8520 Erlangen (DE)**

ACTORUM AG

Röntgengerät zur Herstellung von Transversalschichtbildern und Röntgenschattenbildern eines Aufnahmeobjektes

Die Erfindung betrifft ein Röntgengerät zur Herstellung von Transversalschichtbildern und Röntgenschattenbildern eines Aufnahmeobjekts mit einer Patientenliege, mit einer Strahlenmessanordnung, die eine Strahlenquelle, die ein das Aufnahmeobjekt durchdringendes, in der Schichtebene fächerförmiges Strahlenbündel erzeugt, dessen Querschnittsausdehnung senkrecht zur Schichtebene gleich der Schichtstärke ist und einen Strahlenempfänger, der aus einer quer zur Liegenlängsrichtung verlaufenden Detektorreihe besteht und der die Strahlungsintensität hinter dem Objekt ermittelt, aufweist, mit einer Drehvorrichtung für die Messanordnung zur Drehung um eine parallel zur Längsachse der Patientenliege verlaufende Achse und mit einem Messwertumformer für die Transformation der vom Strahlenempfänger gelieferten Signale in ein Schichtbild, das Mittel zur Relativverschiebung des Strahlenbündels mit Bezug auf die Patientenliege in Längsrichtung der Liege zur Abtastung mehrerer, etwa paralleler Transversalschichten und zur Wiedergabe eines Röntgenschattenbildes aufweist, dessen Länge dem Abstand zwischen den beiden äusseren Transversalschichten und dessen Breite der Breite des Strahlenempfängers entspricht.

Ein Schichtgerät dieser Art ist in der DE-A-2 613 809 beschrieben. Bei diesem bekannten Schichtgerät ist es möglich, zwei Arten von Aufnahmen anzufertigen, nämlich einmal durch Drehung des Röntgenstrahlenbündels in einer Ebene und Erzeugung von Ausgangssignalen des Strahlenempfängers bei verschiedenen Projektionen ein Transversalschichtbild, ein sogenanntes Computertomogramm, und zum anderen durch eine Relativbewegung zwischen der Messanordnung und der Patientenliege bei gegen Drehung verriegelter Messanordnung ein einer üblichen Röntgenaufnahme ähnliches Bild, nämlich ein Röntgenschattenbild. Für die Erzeugung eines Röntgenschattenbildes ist dabei eine mechanische Bewegung entweder der Patientenliege oder der gesamten Messanordnung erforderlich, denn durch den Strahlenempfänger wird immer nur eine Zeile dieses Röntgenschattenbildes erfasst.

In der DE-A-1 941 433 ist ein Röntgengerät zur Herstellung von Transversalschichtbildern eines Aufnahmeobjekts mit einer Patientenliege, mit einer Strahlenmessanordnung, die eine Strahlenquelle, die ein das Aufnahmeobjekt durchdringendes, in der Schichtebene fächerförmiges Strahlenbündel erzeugt, und einen Strahlenempfänger, der aus einer quer zur Liegenlängsrichtung verlaufenden Detektorreihe besteht und der die Strahlungsintensität hinter dem Objekt ermittelt, aufweist, bekannt, das mit einer Drehvorrichtung für die Messanordnung zur Drehung um eine parallel zur Längsachse der Patientenliege verlaufende Achse und mit einem Messwertumformer für die Transformation der vom Strahlenempfänger gelieferten Signale in ein Schichtbild versehen ist. Bei diesem bekannten Röntgengerät ist die für die laterale Abtastung des Aufnahmeobjekts in der untersuchten Schicht erforderliche mechanische Bewegung dadurch reduziert, dass anstelle eines einzigen Detektors als Strahlenempfänger in der geschilderten Weise eine Detektorreihe vorgesehen ist. Es sind jedoch keine Röntgenschattenbilder mit diesem bekannten Röntgengerät erzeugbar, sondern die DE-A-1 941 433 befasst sich nur mit der Herstellung von Transversalschichtbildern, sogenannten Computertomogrammen.

Der Erfindung liegt die Aufgabe zugrunde, ein Schichtgerät der eingangs genannten Art, einen sogenannten Computertomographen, so auszubilden, dass bei ortsfestem Strahlenempfänger und ortsfester Patientenliege das Röntgenschattenbild erzeugt werden kann, so dass sich ein einfacher Aufbau des Gerätes, insbesondere hinsichtlich der dem Strahlenempfänger nachgeschalteten Verarbeitungselektronik, ergibt.

Diese Aufgabe ist erfindungsgemäss dadurch gelöst, dass jeder Detektor des Strahlenempfängers in Liegenlängsrichtung eine Längenausdehnung hat, die der Länge des aufzunehmenden Röntgenschattenbildes entspricht, und dass Mittel vorhanden sind, durch die das Röntgenstrahlenbündel in Liegenlängsrichtung relativ zum Strahlenempfänger bewegbar ist. Bei dem erfindungsgemässen Computertomographen genügt eine Relativbewegung zwischen dem Röntgenstrahlenbündel und dem Strahlenempfänger bei ruhender Patientenliege und ruhendem Strahlenempfänger zur Erzeugung eines Schattenbildes. Diese Bewegung des Röntgenstrahlenbündels kann mit Hilfe von Blenden oder durch eine Bewegung der Röntgenröhre erzeugt werden.

Die Erfindung ist nachfolgend anhand eines in der Zeichnung dargestellten Ausführungsbeispieles näher erläutert. Es zeigen:

Fig. 1 die für die Erfindung wesentlichen Teile eines Schichtgerätes, und

Fig. 2 und 3 Einzelheiten des Gerätes gemäss Fig. 1.

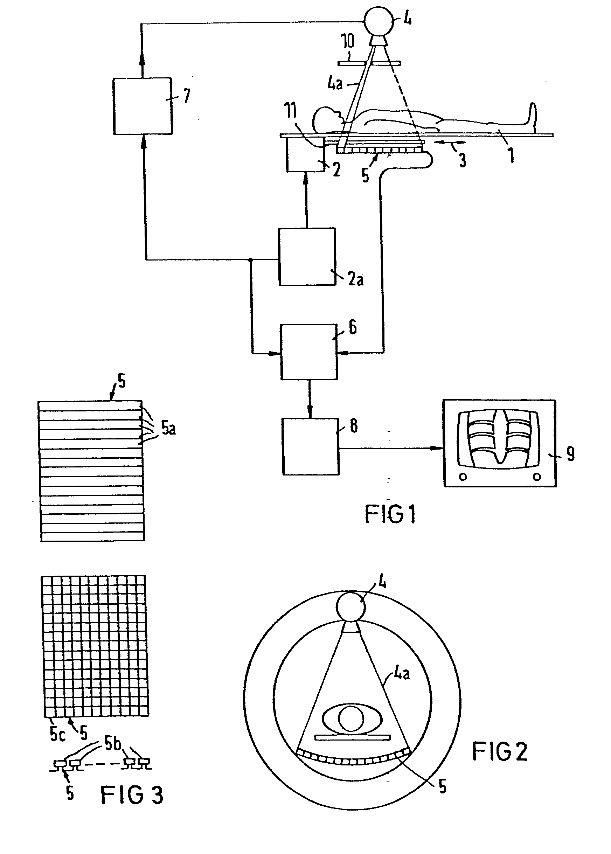

In der Fig. 1 ist eine Patientenliege 1 dargestellt, die durch einen Motor 2 in Richtung des Doppelpfeiles 3 hin- und herbewegbar ist. Zur Erzeugung von Röntgenbildern ist eine Strahlenmessanordnung vorhanden, die aus einer Röntgenröhre 4 und einem Strahlenempfänger 5 besteht. Der Ausgang des Strahlenempfängers 5 ist an einer Messwertabfrageeinheit 6 angeschlossen, die einen Röntgengenerator 7 und einen Rechner 8 steuert. Der Ausgang des Rechners 8 ist an einem Fernsehsichtgerät 9 angeschlossen. Ferner ist eine Steuervorrichtung 2a für den Liegenmotor 2 vorhanden, die auch am Röntgengenerator 7 und an der Messwertabfrageeinheit 6 angeschlossen ist. Die Messanordnung 4, 5 besteht gemäss Fig. 2 aus einer Röntgenröhre 4, welche ein quer zur Längsrichtung der Patientenliege 1 fächerförmiges Röntgenstrahlenbündel 4a erzeugt, das auf dem um eine durch den Fokus der Röntgenröhre 4 parallel zur Längsrichtung der Patientenliege 1 liegende Achse gekrümmten Strahlenempfänger 5 auftrifft. Im Grenzfall kann die Krümmung Null sein; der Strahlenempfänger ist dann gerade.

Der Strahlenempfänger 5 kann aus einer Reihe von langgestreckten Detektoren 5a bestehen, die senkrecht zur Längsrichtung der Patientenliege angeordnet sind (Fig. 3). Wesentliches Merkmal ist die Länge der Detektoren, die der Länge des aufzunehmenden Röntgenschattenbildes entspricht. Die Wirkung eines langen Detektors ist durch eine Reihenschaltung von Detektorelementen 5b kleinerer Länge erzielbar. In diesem Fall besteht der Strahlenempfänger aus einer Vielzahl paralleler, parallel zur Längsrichtung der Patientenliege 1 angeordneter Reihenschaltungen von Detektorelementen 5b. In jeder Reihenschaltung ist eine Vielzahl von Detektorelementen 5b angeordnet.

Zur Einblendung des Röntgenstrahlenbündels 4a in Längsrichtung der Patientenliege 1 sind zwei Schlitzblenden 10 und 11 vorhanden, zwischen denen der Patient 1 liegt. Durch diese Schlitzblenden wird für die Anfertigung eines Computertomogramms das Röntgenstrahlenbündel 4a in Längsrichtung der Patientenliege 1 so eingeblendet, dass es senkrecht zur Längsrichtung der Patientenliege 1 verläuft und eine schmale Schicht des Patienten durchstrahlt sowie bei Verwendung einer Reihenschaltung von Detektorelementen 5b pro Detektor nur eine senkrecht zur Längsrichtung der Patientenliege 1 liegende Detektorelementenreihe des Strahlenempfängers 5 von Röntgenstrahlung getroffen wird. Anschliessend wird die Einheit 4, 5 um die Längsachse des Patienten gedreht. Die in vorbestimmten Stellungen der Messanordnung 4, 5 durch Pulsen der Röntgenröhre 4 gelieferten Ausgangssignale der aktiven Detektorreihe des Strahlenempfängers 5 werden von der Messwertabfrageeineit 6 dem Rechner 8 zugeführt, der daraus ein Transversalschichtbild berechnet und seine Wiedergabe auf dem Fernsehsichtgerät 9 bewirkt.

Zur Herstellung eines Röntgenschattenbildes eines durch die Abmessung des Strahlenempfängers 5 in Längsrichtung der Patientenliege 1, also durch die Anzahl der Detektorelemente pro Reihenschaltung oder die Länge eines Detektors bestimmten Bereiches des Patienten werden der Strahlenempfänger 5 und die Patientenliege 1 in einer vorbestimmten Stellung arretiert, so dass der Strahlenempfänger 5 hinter dem abzubildenden Bereich des Patienten liegt. Anschliessend werden die Schlitzblenden 10 und 11 in Längsrichtung der Patientenliege 1 synchron zueinander zur Bewegung des Strahlenfächers 4a so verstellt, dass bei Anwendung einer Detektorreihe mit langen Detektoren deren unterschiedliche Oberflächenteile in Längsrichtung oder sämtliche Detektorelemente 5b des Strahlenempfängers 5 nacheinander von Röntgenstrahlung getroffen werden, dass also der Strahlenempfänger 5 zeilenweise durch Röntgenstrahlung abgetastet wird. Dabei kann die Röntgenröhre 4 zum Abtasten der einzelnen Zeilen gepulst werden. Man erhält somit vom Strahlenempfänger 5 Ausgangssignale, die das Strahlenschwächungsprofil des vor dem Strahlenempfänger 5 liegenden Bereiches des Patienten wiedergeben. Die Messwertabfrageeinheit 6 gibt diese Signale an den Rechner 8 weiter, der ein übliches Röntgenschattenbild bestimmt und seine Wiedergabe auf dem Fernsehsichtgerät 9 bewirkt.

Aus der Fig. 3 ergibt sich, dass der Strahlenempfänger 5 eine Reihe (ein Array) von langgestreckten Detektoren 5a oder eine Matrix von Detektorelementen 5b darstellt, die zeilenweise vom Röntgenstrahlenbündel 4a, und zwar von der aus dem Patienten austretenden Strahlung abgetastet wird. Jede Bildzeile des auf dem Fernsehsichtgerät 9 wiedergegebenen Bildes entspricht dabei einem Ausschnitt über die langen Detektoren 5a quer zur Liegenlängsrichtung oder einer quer zur Liegenlängsrichtung verlaufenden Reihenanordnung 5c von Detektorelementen 5b. Die Anzahl der Bildpunkte pro Bildzeile ist gleich der Anzahl der Detektorelemente 5b pro Reihenanordnung 5c. Bei dem Beispiel besitzt jede Reihenanordnung 5c fünfzehn Detektorelemente 5b und es sind elf Reihenanordnungen 5c vorhanden. In der Praxis sind diese Zahlen jedoch zur Erzielung einer guten Bildauflösung wesentlich höher.

Der Rechner 8 enthält einen Speicher, der die einer Bildzeile entsprechenden, vom Strahlenempfänger 5 gelieferten Signale jeweils speichert. Nach einer vollständigen Abtastung des Strahlenempfängers 5 in Längsrichtung der Patientenliege 1 ist somit im Rechner 8 das gesamte Röntgenschattenbild gespeichert.

Es ist möglich, die Blende 10 ortsfest in bezug auf die Röntgenröhre 4 anzuordnen, wenn sie zusammen mit dieser in Längsrichtung der Patientenliege 1 verschiebbar angeordnet wird. Auch in diesem Fall ist eine zeilenweise Abtastung des Strahlenempfängers 5 möglich. Hierzu werden die Röntgenröhre 4 und die Blenden 10 und 11 synchron miteinander verstellt.

Für jede Reihe von Detektorelementen kann ein einziger Verstärker zur Messwertverstärkung bei hintereinandergeschalteten Detektorelementen vorgesehen sein. Es ist aber auch möglich, für jedes Detektorelement einen gesonderten Verstärker vorzusehen.

**Patentansprüche**

1. Röntgengerät zur Herstellung von Transversalschichtbildern und Röntgenschattenbildern eines Aufnahmeobjekts mit einer Patientenliege (1), mit einer Strahlenmessanordnung, die eine Strahlenquelle (4), die ein das Aufnahmeobjekt durchdringendes, in der Schichtebene fächerförmiges Strahlenbündel (4a) erzeugt, dessen Querschnittsausdehnung senkrecht zur Schichtebene gleich der Schichtstärke ist und einen Strahlenempfänger (5), der aus einer quer zur Liegenlängsrichtung verlaufenden Detektorreihe besteht und der die Strahlungsintensität hinter dem Objekt ermittelt, aufweist, mit einer Drehvorrichtung für die Messanordnung (4, 5) zur Drehung um eine parallel zur Längsachse der Patientenliege (1) verlaufende Achse und mit einem Messwertumformer (6, 8, 9) für die Transformation der vom Strahlenempfänger gelieferten Signale in ein Schichtbild, das Mittel (10) zur Relativverschiebung des Strahlenbündels mit Bezug auf die Patientenliege (1) in Längsrichtung der Liege zur Abtastung mehrerer, etwa paralleler Transversalschichten und zur Wiedergabe eines Röntgenschattenbildes aufweist, dessen Länge dem Abstand zwischen den beiden äusseren Transversalschichten und dessen Breite der Breite des Strahlenempfängers (5) entspricht, dadurch gekennzeich-

net, dass jeder Detektor (5a) des Strahlenempfängers (5) in Liegenlängsrichtung eine Längenausdehnung hat, die der Länge des aufzunehmenden Röntgenschattenbildes entspricht, und dass Mittel (10) vorhanden sind, durch die das Röntgenstrahlenbündel (4a) in Liegenlängsrichtung relativ zum Strahlenempfänger (5) bewegbar ist.

2. Schichtgerät nach Anspruch 1, dadurch gekennzeichnet, dass jeder Detektor des Strahlenempfängers (5) in Liegenlängsrichtung aus der Reihenschaltung einer Vielzahl von Detektorelementen (5b) besteht, so dass der Strahlenempfänger (5) von einer Matrix aus Detektorelementen (5b) gebildet ist.

3. Röntgengerät nach Anspruch 1 oder 2, dadurch gekennzeichnet, dass zur Bewegung des Röntgenstrahlenbündels (4a) in der Längsrichtung der Patientenliege (1) zwei Schlitzblenden (10, 11) vorhanden sind, zwischen die der Patient einschiebbar ist und die synchron zueinander zur Bewegung des Strahlenfächers (4a) im Sinne einer zeilenweisen Abtastung des Strahlenempfängers (5) verstellbar sind.

4. Röntgengerät nach Anspruch 1 oder 2, dadurch gekennzeichnet, dass zur Bewegung des Röntgenstrahlenbündels (4a) die Strahlenquelle (4) zusammen mit einer unter der Patientenliege (1) angeordneten Schlitzblende (11) derart verstellbar ist, dass der Strahlenempfänger (5) durch den Strahlenfächer (4a) zeilenweise abgetastet wird.

## Claims

1. An X-ray device for the production of transversal planigraphs and X-ray shadow images of an object to be photographed, comprising a patient's bed (1), a radiation measuring arrangement having a radiation source (4) generating a beam (4a) which penetrates the object to be photographed, is fan-shaped in the stratification plane and whose cross-sectional dimension at right angles to the stratification plane is equal to the layer thickness, and a radiation receiver (5) which consists of a row of detectors running transversely to the longitudinal direction of the bed and which determines the radiation intensity behind the object, a rotation device for the measuring arrangement (4, 5) which effects a rotation about an axis running parallel to the longitudinal axis of the patient's bed (1) and a measured value transformer (6, 8, 9) for the transformation of the signals supplied by the radiation receiver into a planigraph, which possesses means (20) for effecting a relative shift of the beam relative to the patient's bed (1) in the longitudinal direction of the bed in order to scan a plurality of approximately parallel transversal planes and in order to reproduce an X-ray shadow image, the lenght of which corresponds to the distance between the two outer transversal planes, and the width of which corresponds to the width of the radiation receiver (5), characterised in that in the longitudinal direction of the bed each detector (5a) of the radiation receiver (5) has a longitudinal dimension which corresponds to the length of the X-ray shadow image to be recorded, and means (10) are provided by which the X-ray beam (4a) can be moved in the longitudinal direction of the bed relative to the radiation receiver (5).

2. An X-ray device as claimed in Claim 1, characterised in that in the longitudinal direction of the bed each detector of the radiation receiver (5) consists of the series arrangement of a plurality of detector elements (5b) so that the radiation receiver (5) is formed by a matrix of detector elements (5b).

3. An X-ray device as claimed in Claim 1 or 2, characterised in that for the X-ray beam (4a) to be moved in the longitudinal direction of the patient's bed (1) there are arranged two slotted diaphragms (10, 11) between which the patient can be inserted and which can be adjusted synchronously to one another in order to move the fan-shaped beam (4a) in the sense of a row-by-row scanning of the radiation receiver (5).

4. An X-ray device as claimed in Claim 1 or 2, characterised in that for the X-ray beam (4a) to be moved, the radiation source (4) together with a slotted diaphragm (11) arranged beneath the patient's bed (1) can be adjusted in such manner that the radiation receiver (5) is scanned row-by-row by the fan-shaped beam (4a).

## Revendications

1. Appareil à rayons X pour la préparation de tomographies et radioscopies d'un objet de prise de vue, avec une surface de repos (1) pour le patient, avec un dispositif de mesure du rayonnement qui comporte une source de rayonnement (4) produisant un faisceau de rayons (4a) traversant l'objet de la prise de vue et ayant la forme d'un éventail dans le plan de la couche, et dont l'extension de la section transversale, perpendiculaire au plan de la couche, est égale à l'épaisseur de la coche, ainsi qu'un récepteur de rayonnement (5) qui est constitué par une rangée de détecteurs s'étendant transversalement à la direction longitudinale de la surface de repos et qui détermine l'intensité du rayonnement derrière l'objet, avec un dispositif de mise en rotation pour le dispositif de mesure (4, 5) en vue d'une rotation autour d'un axe s'étendant parallèlement à l'axe longitudinal de la surface de repos (1) pour le patient, et avec un convertisseur de valeur de mesure (6, 8, 9) pour la transformation des signaux fournis par le récepteur de rayonnement en une tomographie, qui présente des moyens (10) pour le déplacement relatif du faisceau de rayonnement par rapport à la surface de repos (1) pour le patient, en direction longitudinale de la surface de repos, en vue du balayage de plusieurs couches transversales sensiblement parallèles et de la restitution d'une radioscopie, dont la longueur correspond à la distance entre les deux couches transversales extérieures et dont la largeur correspond à la largeur du récepteur de rayonnement (5), caractérisé par le fait que chaque détecteur du récepteur de rayonnement (5) a, dans la direction longitudinale de la surface d'appui, une extension longitudinale qui correspond à la longueur de la radioscopie à établir, et que des moyens (10) sont prévus à l'aide desquels le faisceau de rayons X (4a) est susceptible d'être déplacé dans la direction longitudinale de la surface d'appui, par rapport au récepteur de rayonnement (5).

2.   Tomographe selon la revendication 1, caractérisé par le fait que chaque détecteur du récepteur de rayonnement (5) est constitué, dans la direction longitudinale de la surface de repos, par un montage série d'une pluralité d'éléments-détecteurs (5b) en sorte que le récepteur de rayonnement (5) est formé par une matrice d'éléments-détecteurs (5b).

3.   Apparail à rayons X selon la revendication 1 ou 2, caractérisé par le fait que pour déplacer le faisceau de rayons X (4a) dans la direction longitudinale de la surface de repos (1) du patient, il est prévu deux diaphragmes à fentes (10, 11) entre lesquels on peut faire glisser le patient, diaphragmes qui sont susceptibles d'être déplacés en synchronisme entre eux, en vue du déplacement du rayonnement en éventail (4a) dans le sens d'un balayage, ligne par ligne, du récepteur de rayonnement (5).

4.   Appareil à rayons X selon la revendication 1 ou 2, caractérisé par le fait que pour déplacer le faisceau de rayons X (4a) la source de rayonnement (4) est susceptible de déplacer, avec un diaphragme à fentes (11) disposé sous la surface de repos (1) du patient, de manière que le récepteur de rayonnement (5) soit balayé, ligne par ligne, par le faisceau en forme d'éventail (4a).

FIG 1

FIG 2

FIG 3